# EUROPEAN PATENT SPECIFICATION

(11) **EP 1 776 937 B1**
(45) Date of publication and mention of the grant of the patent: **24.06.2009**
(21) Application number: 06122295.6
(22) Date of filing: 13.10.2006
(51) Int. Cl.: A61F 2/46, A61F 2/36

(54) **Tool**
Werkzeug
Outil

(30) Priority: 18.10.2005 GB 0521173
(43) Date of publication of application: 25.04.2007
(73) Proprietor: Finsbury (Development) Limited, Leatherhead, Surrey (GB)
(72) Inventor: Tuke, Michael Antony, Guildford, Surrey GU1 3TF (GB)
(74) Representative: Smaggasgale, Gillian Helen

(56) References cited:
- EP-A- 1 477 120
- DE-U1- 7 601 139
- DE-U1- 20 213 728

## Description

The present invention relates to a tool for use in hip resurfacing operations. More particularly, it relates to a tool for assisting in the insertion of a replacement femoral head.

The efficient functioning of the hip joints is extremely important to the well being and mobility of the human body. Each hip joint is comprised by the upper portion of the upper leg bone (femur) which terminates in an offset bony neck surmounted by a ball-headed portion which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component of the prosthesis can then be inserted into place. In some arrangements, the acetabular component may simply be held in place by a tight fit with the bone. However, in other arrangements, additional fixing means such as screws or bone cement may be used. The use of additional fixing means help to provide stability in the early stages after the prosthesis has been inserted. In some modem prosthesis, the acetabular component may be coated on its external surface with a bone growth promoting substance which will assist the bone to grow and thereby assist the holding of the acetabular component in place. The bone femoral head will be removed and the femur hollowed using reamers and rasps to accept the prosthesis. The stem portion will then be inserted into the femur.

In some cases, a femoral component of the kind described above may be replaced with components for use in femoral head resurfacing or for use in thrust plate technology. In femoral head resurfacing the ball-head of the femur may be machined to accept a femoral head resurfacing prosthesis. In particular, in a generally practiced procedure, the top of the head of the femur is partially resected using a saw and a well is drilled into the resected head to accept the stem of the femoral head resurfacing prosthesis. The drilling of the well may occur before or after the top of the head is partially or fully resected. A sleeve cutter is then used to machine the head of the femur. A chamfering tool with then generally be used to further shape the femoral head. The femoral head is then ready to receive the femoral head resurfacing prosthesis in which the stem of the prosthesis is inserted into the well.

It will be acknowledged that it is essential that the replacement surface for the head of the femur should be precisely located in both angular and translation positions of the axis of the femoral neck of the implant. To assist this, in some techniques, the surgeon inserts a pin in the lateral femur. The desired position of the pin will be known from pre-operative analysis of the x-rays. The surgeon will measure the desired distance down the femur from the tip of the greater trochanter and the alignment pin is inserted through the vastus lateralis fibres. The alignment pin is inserted in a transverse direction into the mid-lateral cortex and directed upwardly towards the femoral head. The pin is left protruding so that an alignment guide can be hooked over the alignment pin. Suitable alignment guides include those known as the McMinn Alignment Guide available from Midland Medical Technologies Ltd.

Alignment guides of the kind described above generally comprise a hook or aperture which is placed over the alignment pin thus providing a good angular position for the axis of the implant in valgus, varus and ante-version of the neck. The guide will then be adjusted so that a cannulated rod is located such that the aperture therein is directed down the mid-lateral axis of the femoral neck. A stylus having been set to the desired femoral component size is positioned such that it can be passed around the femoral neck. When the stylus can be passed around the femoral neck, the cannulated rod is locked in position. Once the guide is stabilised in this way fine adjustments can be made until the surgeon is happy that the guide is in the required position.

A guide wire can then be inserted though the cannulated rod. This guide wire is then used in the further surgery in which the femoral head is shaped to accept the prosthesis. It will be understood that the alignment guide is an essential tool in the surgical procedure to ensure that the aperture drilled in the femoral head is both central to the femoral neck and at the correct angle of alignment to the femoral neck and that the shaping of the femoral head is accurate for the chosen head size.

Failure to position the alignment guide correctly may have the disastrous effect of allowing the machining of the cylinder of the head during the shaping procedure to "notch" into the neck of the femur. This will predispose the bone to early failure on load bearing.

Other guides are known which are, in use, located on the femoral neck itself. These are used in a similarmanner to those described above and may involve some adjustment by the surgeon before he selects the best position.

Whilst the prior art alignment guides are particularly suitable for their function and have reached a high level of acceptance amongst surgeons, there is now a move towards a less invasive surgical technique in which the required incision is as small as possible and the amount of interaction with healthy tissue is minimised. It is therefore desirable to consider carrying out femoral head resurfacing without the need to insert the alignment pin so that all of the surgical procedure takes place at the femoral head. Similarly it is desirable to be able to apply a thrust plate prosthesis without the need to pre-insert the alignment pin. There is therefore a requirement for an alignment guide which can function without interaction with an alignment pin and one example of a suitable alignment guide of this kind can be seen in EP 1588668. The alignment guide of EP 1588668 is particularly useful for ensuring that the well is drilled in the correct orientation.

Once the well has been formed, the head resurfacing prosthesis can be located in position. In order to achieve this the stem of the femoral head resurfacing prosthesis is placed into the well in which it is generally a sliding fit. Since it is essential that the prosthesis is held firmly in place, the stem, whilst being a sliding fit, the cross-section of the well will generally be selected such that the stem of the prosthesis is a tight fit in the well. Thus force is required to insert the prosthesis into its position. Generally it is necessary to apply force to the surface of the prosthesis. Although this force may be applied by striking the prosthesis with a hammer or other similar tool, an introducer tool will generally be used in which a striking surface is connected to a handle which has a lower surface which sits on the outer surface of the prosthesis.

Whilst sufficient force to insert the prosthesis can be provided by striking with a tool of this type, it is difficult to control the amount of force applied. In addition, the use of a tool which is separate from the prosthesis means that it is difficult to ensure that the force is applied in the optimum direction and it is possible that a glancing blow may be applied. If the force is applied in other than the optimum direction or is too hard, there is a risk that the machined head of the femur may be damaged or the force passing through the femur will damage the femoral neck. In extreme cases, the neck of the femur may be fractured or weakened to the extent that fracture occurs later.

A further problem with applying the force by means of a series of blows is that a pressure may build up in, for example, blood vessels which can lead to embolisms. Similarly deposits in veins etc may be dislodged which can also result in embolisms being formed.

In order to ensure that the prosthesis is held firmly in place, cement will generally be placed into the underside of the head resurfacing prostheses prior to the insertion of the stem into the well in the femur such that bonding can occur between the machined head of the femur and the femoral head resurfacing prosthesis. However, the presence of the cement increases the problems encountered in seating the prosthesis in the correct position since as the stem of the prosthesis is inserted into the well, the adhesive provides resistance to the seating of the head in the correct position, thereby requiring the application of still further force.

There is therefore a need to provide a tool which enables the force required to apply the head resurfacing prosthesis to a prepared femoral head to be controlled and applied in the required optimum direction.

It has been found that the problems detailed above can be solved by the provision of a jig which interacts with the femur and allows the force to be applied in a manner whereby the movement can be regarded as a squeezing motion.

Thus according to a first aspect of the present invention there is provided a jig for the application of a femoral head resurfacing prosthesis to a prepared femoral head wherein the jig comprises:
at least one support arm having a proximal end, a distal end and a plane defined therein;
a jaw located at the distal end of the support arm and defining a plane, wherein the plane of the jaw is angled to the plane of the support arm; and
a static member located at the proximal end of the or each support arm;
characterized in that it further comprises an applicator member associated with the static member and movable with respect thereto; and
a connector means to enable the applicator member in use to interact with an outer surface of a femoral head resurfacing prosthesis;
the applicator member comprising operating means to enable the applicator member to be moved from a first upper position to a second lower position.

It will be understood that in use, the jaw can be placed around the femur. It will generally be placed around the neck of the femur immediately behind the femoral head but it will be understood that any suitable location may be used.

The jig of the present invention offers at least two advantages over prior art arrangements. First, the positioning of the jig in which the jaw is located on the femur will ensure that the pressure applied to the femoral head resurfacing prosthesis as the applicator member is moved from the first position to the second position is applied in the optimum direction and orientation.

The second benefit is that the location of the jaw serves as a resist to the application of the force required to move the applicator member from a first upper position to a second lower position such that the force applied to the prosthesis can be controlled in magnitude. The presence of the resist also assists the force to be applied in a smooth controlled manner rather than the sudden jarring force which may be experienced when the prior art arrangements are used.

The jaw of the present invention may be a ring. The ring will generally be angled at between 80° and 100° to the plane of the support arm and more preferably will be substantially perpendicular to the support arm.

The jaw may be positioned such that the centre of the applicator member is in line with the centre point of the ring. Alternatively, the centre point may be offset from the axis of the bore. In one arrangement, the jig may include means to enable the user to adjust the position of the ring relative to the axis of the bore.

The jig is configured such that in use the jaw may be located beneath the head of the femur at the neck-head junction. Any suitable size of jaw may be used. However, the size will generally be selected to closely interact with the femur. However, in one arrangement, the jaw may of a larger size and a detachable collar may be included which connects to the jaw. A particular advantage of using the detachable collar is that if any excess cement is extruded from underneath the prosthesis as it is applied it is caught by the collar. It will therefore be understood that it is particularly advantageous that the collar is a disposable collar. The collar may be formed from any suitable material but will generally be formed from a rigid plastics material. The collar may be of any suitable configuration. However, where the jaw is a ring, the collar may be a split ring.

Where the jaw is a ring it may be of any suitable configuration. In one arrangement, the ring is a C-ring so that in use the guide can be placed in position by passing the neck of the femoral head through the open space in the ring. It will be understood that the size and position of the break in the C-ring may mean that the ring may be placed around the femoral neck in one orientation and then the device rotated to the desired position for surgery.

In another arrangement the ring may have a portion which is hinged to the remainder of the ring and which in an open orientation will enable the guide to be placed in position around the neck of the femoral head. The hinged portion can then be closed to complete the ring. Thus the hinged portion may be regarded as a gate or door.

In a still further arrangement the ring may be formed of two arms which are each hinged to allow movement to an open position such that the guide may be located in position. When in the closed position in use, the two arms will close around the neck of the femur. In one arrangement, the two arms will be sized such that when in the closed position, they complete a circle but they may simply form a segment of a circle. The two arms may be of the same or different lengths. Thus where the arms, when in the closed position, complete a circle, the two arms may meet at a point opposite the point of connection to the support arm or may be at another point on the circumference of the circle. In one alternative arrangement one arm may be fixed and the other may be adjustable thereto.

In a further alternative arrangement, the ring may be adjustable. For example, an iris which will expand to enable the ring to be passed over the femoral head and then contracted to fit around the neck of the femur may be used.

In one arrangement, a range of jigs may be provided each with a different size ring permanently attached thereto. However, in one alternative arrangement, the ring may be demountable so that different size rings may be used as dictated by the size of the patient's femur. According to a second aspect of the present invention there is provided a kit comprising the jig of the above first aspect and a plurality of rings.

Where separate rings are provided, they may be provided with a peg extending upwardly from the ring which engages with the support arm in any suitable manner. In one arrangement, the peg may be shaped to be a sliding fit in a channel in the support arm. In an alternative arrangement, the peg may be of tubular cross-section and may when connected to the support arm extend around the support arm. The peg may be fastened to the support arm by any suitable means. In one arrangement, a locking means, such as a screw lock, may be provided.

It will be understood that the ring component is preferably chosen to fit onto the head-neck junction of the femur once bony protuberances such as osteophytes have been removed.

It will be acknowledged that the ring, whether a full or part circle such as the C-ring, does not have to be shaped as at least part of a circle. For example, the ring may be at least part of the portion of another shape such as a triangle, square, pentagon, hexagon, heptagon, octagon or other multi-sided shapes. It may be of a shape similar to that of a horse-shoe. In an alternative arrangement it may be configured to the shape of the femoral neck or the head/neck junction. Thus it should be understood that any reference to "circle" and "ring" should be construed to include these other shapes. "Circle" and "ring" are simply used for ease of reference.

In one alternative arrangement, the jaw is not a ring but is any arrangement which enables the jaw to provide the necessary resistant force. For example, where there is more than one support arm, each may be provided with a foot which interacts with the femur.

The static member located at the proximal end of the or each support arm may be of any suitable configuration. In one arrangement it may be a collar in which the applicator member is movable.

In one arrangement, the applicator member may be a sliding-fit in a collar formed by the static member. In this arrangement the upper end of the applicator member may be provided with operating means which will enable the user to depress the applicator member. In one arrangement, the operating means is a thumb plate which may be located at the upper end of the applicator member. It will be understood that whilst the plate is referred to as a thumb plate, in use the surgeon may use any digit or his hand or even a separate tool to cause the applicator member to move from the first position to the second position. In an alternative arrangement, the plate may be sized such that in use it will fit in the hand of the surgeon's hand.

In the arrangements in which a thumb, or larger plate is used, at least a portion of the support arm may be shaped to provide a comfortable position for at least one of the user's fingers in use. One or more additional handles of any suitable configuration may also be used. The shaped portion of the support arm and any additional handles contribute to the resist force provided by the jig.

In one alternative arrangement, the operating means may be mechanical means used to move the applicator member from the first position to the second position. In one arrangement, the applicator means may be threaded and the internal surface of the static member will then have cooperating threads. In this arrangement, rotation of the applicator member will cause it to move from the first position to the second position. In this arrangement, the operating means will generally be provided at the upper end of the applicator means to assist the user to rotate the applicator member to advance it from the first to the second position. The rotation means will generally be a knob located at the upper end of the applicator means. In a further alternative arrangement, the means for advancing the screw means may be provided on the static member.

In another arrangement, the operating means may be a rack and pinion arrangement provided to enable the applicator means to be advanced from a first position to a second position. In this arrangement, the rack will generally be provided on the applicator means and the pinion element on an internal surface of the static member a handle to enable to the rack and pinion device to be advanced may be present.

In an alternative arrangement, the static member may be shaped such that a sprung applicator member can pass through it and the operating means is a trigger element mounted on the static member which when activated forces the applicator member from the first to the second position.

The connector means will generally be located at the distal end of the applicator means. The connector means may simply be formed by means of the shape of the end of the applicator member. In an alternative arrangement, a plunger may be located on the distal end of the applicator means. In one arrangement the plunger may be formed from a plastics material. The plunger may be disposable.

A flag and/or guide pin may be included at the distal end of the support arm and where present will generally extend below the position of the ring. The flag and/or guide pin may be integral with the support arm or may be provided as a separate fitting. This flag or pin will serve as a visual guide to assist the surgeon to check that the jig is in the correct alignment with the medial side of the femoral neck to ensure the correct valgus-varus alignment and the correct ante-version angles. A second such arrangement of flag and/or pin may be provided to assist in aligning the jig with the ante-version of the femoral neck.

The length of the support arm may be selected such that in use the positioning of the jaw and/or the collar, where present, may represent the position at which the end of the prosthesis will lie when in the correct position thereby providing the surgeon with a visible indicator of when no further force is required to drive the prosthesis downwardly.

The present invention will now be described by way of example with reference to the accompanying figures in which:
- Figure 1: is a side view of the jig of one arrangement of the present invention in use;
- Figure 2: is a side view of an alternative arrangement of the present invention;
- Figure 3: is a side view of a second alternative arrangement of the present invention;
- Figure 4: is a side view of a third alternative arrangement of the present invention;
- Figure 5: is a side view of a fourth alternative arrangement of the present invention;
- Figure 6: is a side view of a fifth alternative arrangement of the present invention;
- Figure 7: is a perspective view of the collar and a cross-section showing the interaction of the collar with the jaw of the jig;
- Figure 8: is a side view of a sixth alternative arrangement of the present invention; and
- Figure 9: is a side view of a seventh alternative arrangement of the present invention

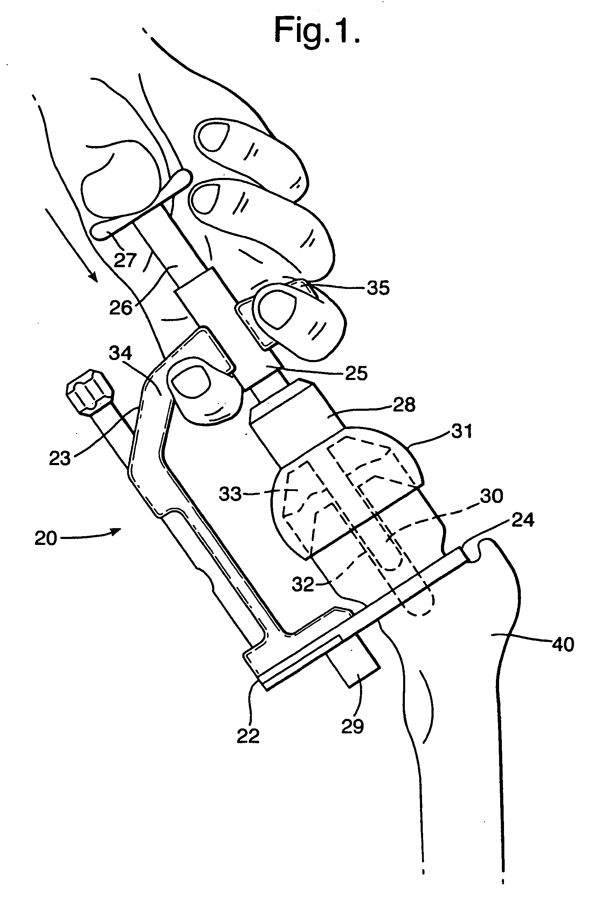

As illustrated in Figure 1, the jig 20 of one embodiment of the present invention comprises a support arm having a distal 22 and a proximal 23 end a ring 24 is located at the distal end of the arm and extends therefrom such that it is at right angles to the plane of the arm. However, it will be noted that the arm is shaped to allow the arm, in use, to fit around the femoral head a static member in the form of a collar 25 located at the proximal end of the support arm. An applicator member 26 is a sliding fit in the collar. A thumb plate 27 is located at the proximal end of the applicator member and a connector means 28 in the form of a plastics plunger is located at the distal end.

A flag 29 may be present to assist the surgeon to visually confirm that the alignment guide is in the desired position.

A shaped region 34 of the support arm and a handle 35 are provided to enable the fingers to be positioned in use.

In use the ring is located around the neck of the femur 40. The stem 30 of the prosthesis 31 is located in a well 32. Cement 33 is present within the prosthesis. The thumb plate 27 can then be depressed to cause the applicator member 26 can be moved from a first upper position to a second lower position thereby forcing the prosthesis into position.

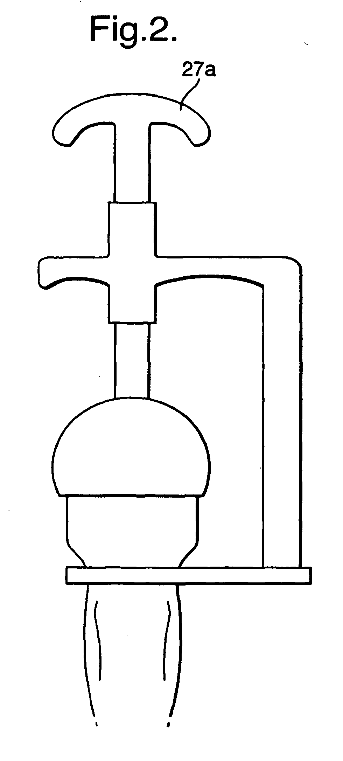

An alternative arrangement is illustrated in Figure 2 the thumb plate may be replaced with a disc 27a which can be placed in the palm of the user's hand.

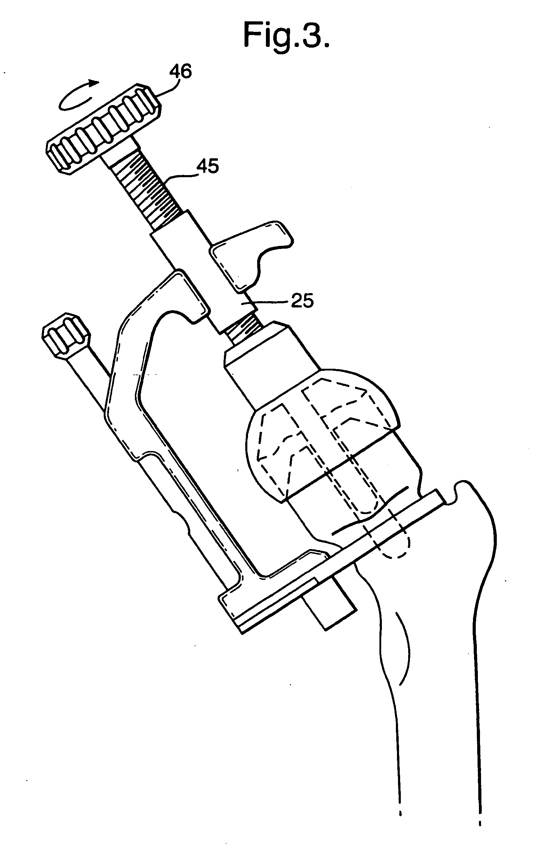

A further alternative arrangement is illustrated in Figure 3. In this arrangement, the applicator member is threaded 45 and a cooperating thread is located on the internal surface of the static member 25. Rotation of the knob 46 enables the applicator member to be moved from the first to the second position.

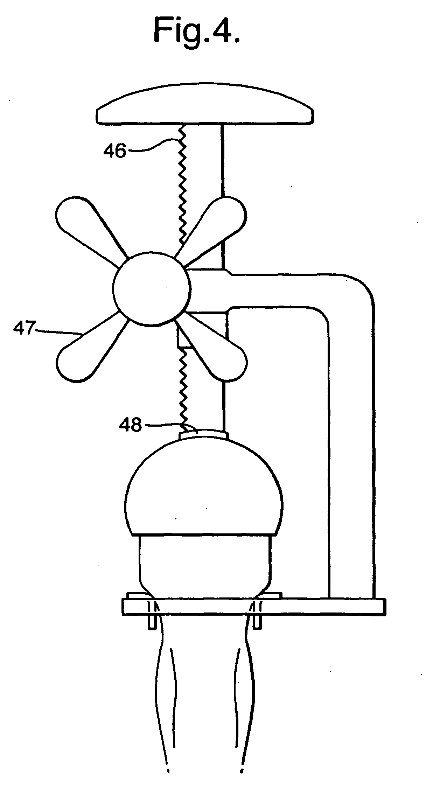

A still further alternative arrangement is illustrated in Figure 4. Here a rack and pinion arrangement 46 may be used. A handle 47 is provided to enable the rack and pinion arrangement to be operated to move the applicator from the first position to the second position. In the illustrated arrangement, the connector means 48 to the prosthesis is provided by a curved shape on the end of the distal end of the applicator means. It will be understood that a plastics plunger or other suitable arrangement may be used.

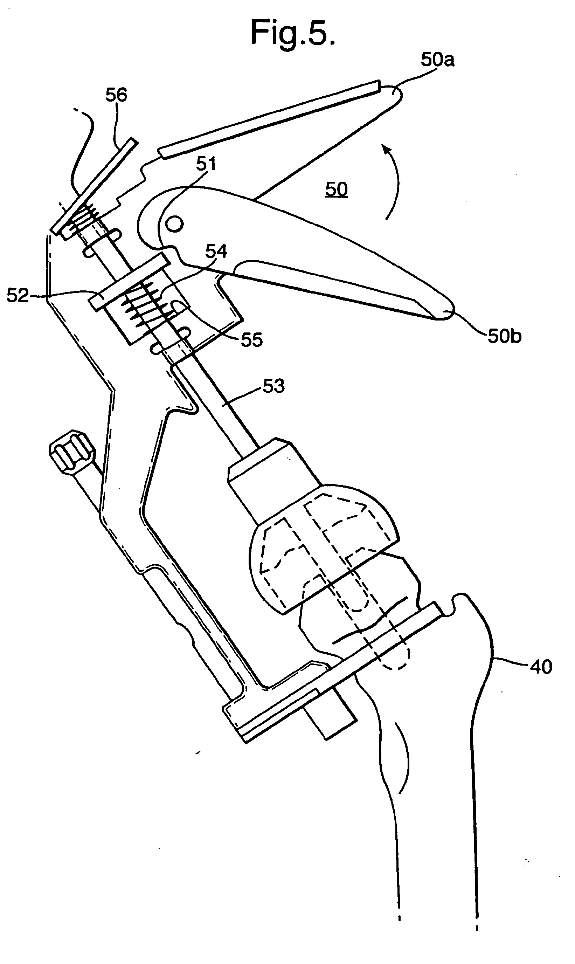

A still further arrangement is illustrated in Figure 5. Here the operating means may be a trigger comprising a fixed handle 50a and a movable handle 50b. In use, as the movable handle 50b is moved towards the fixed handle 50a. The movement causes the cam 51 to interact with a plate 52 connected to the applicator means 53. In this arrangement, the applicator means is a rod 53. A spring 54 is provided to provide resistance between the plate 52 and a portion of the static member 55. A locking member 56 may be provided.

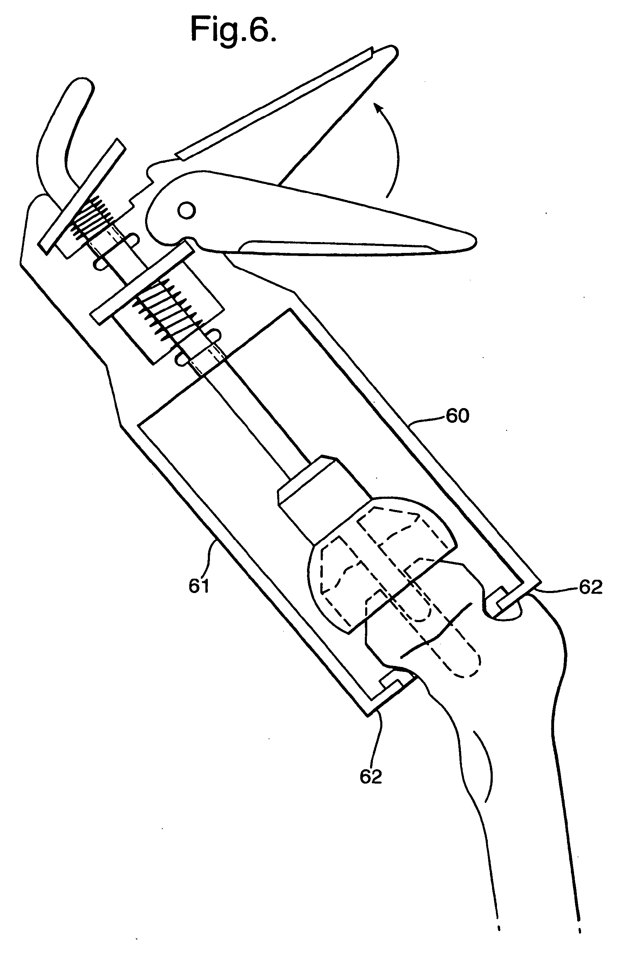

An alterative arrangement is illustrated in Figure 6. In this arrangement, two support arms 60, 61 are provided and a foot 62 is located at the distal end of each arm. The feet provide the jaw of the present invention. Whilst the use of two support arms is illustrated with the arrangement in which the operating means is a trigger, it will be understood that alternative arrangements, including those detailed above may be used.

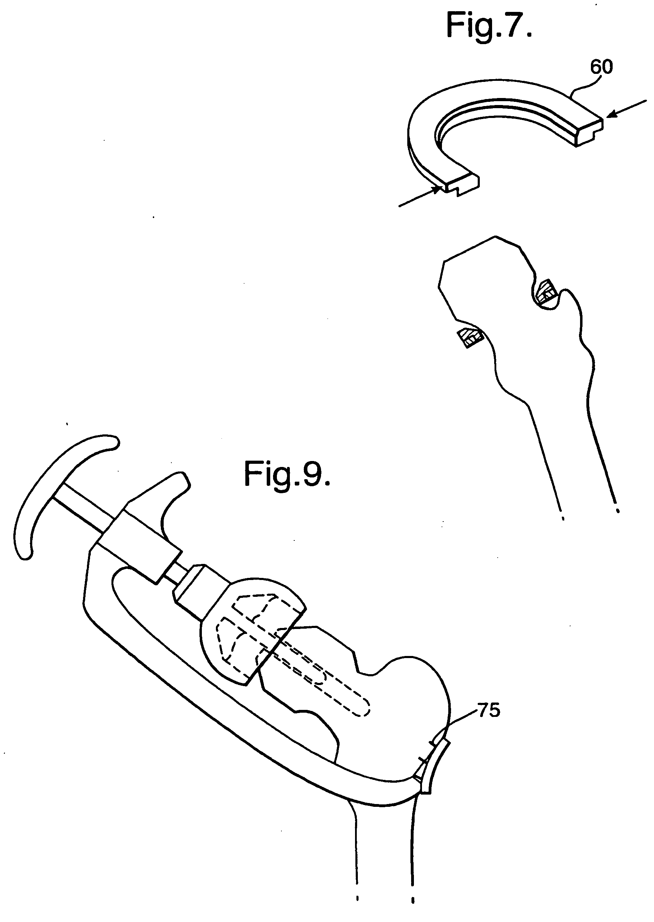

A collar 60 such as the one illustrated in Figure 7 may be used. The collar 60 may be shaped to fit on the jaw of the jig.

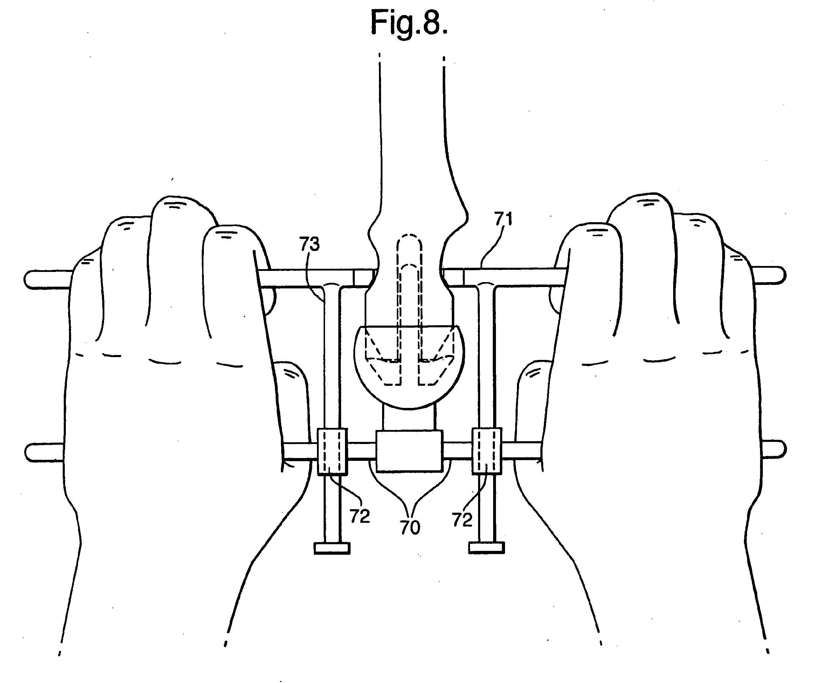

A further embodiment of the present invention is illustrated in Figure 8 in which two handle bar systems are used. In use, the surgeon will use two hands to move the upper handle bar 70 downwardly towards a lower fixed handle bar 71. The upper handle bar has two collars 72 integral therewith which are slidable on the support members 73.

As illustrated in Figure 9, the support arm and the jaw may be configured such that the jaw interacts with the lateral portion of the femur rather than the femoral neck. In this arrangement, the jaw may include teeth 75 to increase friction between the jaw and the bone and prevent slippage. Although the positioning of the jaw against the lateral femur has been illustrated for the arrangement similar to Figure 2, it will be understood that this arrangement of the support arm and jaw may be used with any of the possibilities for the static member and the applicator member described above.

## Claims

1. A jig (20) for the application of a femoral head resurfacing prosthesis (31) to a prepared femoral head wherein the jig comprises:
at least one support arm having a proximal end (23), a distal end (22) and a plane defined therein;
a jaw located at the distal end (22) of the support arm and defining a plane, wherein the plane of the jaw is angled to the plane of the support arm; and
a static member located at the proximal end of the or each support arm;
**characterised in that** it further comprises an applicator member (26) associated with the static member and movable with respect thereto; and
a connector means (28) to enable the applicator member (26) in use to interact with an outer surface of a femoral head resurfacing prosthesis (31);
the applicator member comprising operating means to enable the applicator member (26) to be moved from a first upper position to a second lower position.

2. A jig according to Claim 1 wherein the jaw is a ring.

3. A jig according to Claim 1 or Claim 2 wherein the jaw is located such that it is substantially perpendicular to the support arm.

4. A jig according to Claim 2 wherein the jaw is positioned such that the centre of the applicator member is in line with the centre point of the ring.

5. A jig according to any one of Claims 1 to 4 wherein the jig additionally includes a detachable collar.

6. A jig according to Claim 5 wherein the collar is a split ring.

7. A jig according to any one of Claims 1 to 6 wherein the static member located at the proximal end of the or each support arm is a collar in which the applicator member is movable.

8. A jig according to Claim 7 wherein the applicator member is a sliding-fit in the collar.

9. A jig according to Claim 8 wherein the upper end of the applicator member is provided with operating means to enable the applicator member to be depressed.

10. A jig according to Claim 9 wherein the operating means is a plate.

11. A jig according to Claim 9 or 10 wherein at least a portion of the support arm is shaped to provide a position for at least one of the user's fingers in use.

12. A jig according to any one of Claims 1 to 12 wherein one or more handles is present.

13. A jig according to Claim 1 wherein the operating means is mechanical means.

14. A jig according to Claim 1 wherein the applicator means is threaded and cooperating threads are provided on the static member.

15. A jig according to Claim 1 wherein the operating means is a rack and pinion arrangement.

16. A jig according to Claim 1 wherein the operating means is a trigger mechanism

17. A jig according to any one of Claims 1 to 16 wherein the connector means is formed by the shape of the end of the applicator member.

18. A jig according to any one of Claims 1 to 16 wherein the connector means is a plunger located on the distal end of the applicator means.

19. A jig according to Claim 18 wherein the plunger is formed from a plastics material.

20. A jig according to any one of Claims 1 to 19 wherein the support arm is configured such that the jaw interacts with the femoral neck.

21. A jig according to any one of Claims 1 to 19 wherein the support arm is configured such that the jaw interacts with the femoral neck.

## Patentansprüche

1. Schablone (20) für die Anlage einer Femurkopf-Oberflächenwiederherstellungsprothese (31) an einen präparieren Femurkopf mit
wenigstens einem Tragarm mit einem proximalen Ende (23), einem distalen Ende (22) und einer auf ihm umgrenzten Ebene,
einer an dem distalen Ende (22) des Tragarms angeordneten Backe, die eine Ebene umgrenzt, wobei die Ebene der Backe zu der Ebene des Tragarms abgewinkelt ist, und
einem an dem proximalen Ende des oder jedes Tragarms angeordneten statischen Teil,
**dadurch gekennzeichnet, daß** sie ferner aufweist:
ein mit dem statischen Teil verbundenes, zu diesem bewegliches Applikatorteil (26) und
ein Verbindermittel (28), um bei Benutzung dem Applikatorteil (26) zu ermöglichen, mit der äußeren Fläche einer Femurkopf-Oberflächenwiederherstellungsprothese (31) in Wechselwirkung zu treten,
wobei das Applikatorteil Betätigungsmittel aufweist, um dem Applikatorteil (26) zu ermöglichen, sich aus einer ersten oberen Stellung in eine zweite untere Stellung zu bewegen.

2. Schablone nach Anspruch 1, bei der die Backe ein Ring ist.

3. Schablone nach Anspruch 1 oder Anspruch 2, bei der die Backe so angeordnet ist, daß sie im wesentlichen senkrecht zu dem Tragarm ist.

4. Schablone nach Anspruch 2, bei der die Backe so angeordnet ist, daß die Mitte des Applikatorteils mit dem Mittelpunkt des Ringes ausgefluchtet ist.

5. Schablone nach einem der Ansprüche 1 bis 4, bei der die Schablone ferner eine abnehmbare Manschette hat.

6. Schablone nach Anspruch 5, bei der die Manschette ein gespaltener Ring ist.

7. Schablone nach einem der Ansprüche 1 bis 6, bei der der an dem proximalen Ende des oder jedes Tragarms angeordnete statische Teil eine Manschette ist, in der das Applikatorteil beweglich ist.

8. Schablone nach Anspruch 7, bei der das Applikatorteil mit Gleitsitz in der Manschette ist.

9. Schablone nach Anspruch 8, bei der das obere Ende des Applikatorteils mit einem Betätigungsmittel versehen ist, um das Applikatorteil niederdrücken zu können.

10. Schablone nach Anspruch 9, bei der das Betätigungsmittel eine Platte ist.

11. Schablone nach Anspruch 9 oder 10, bei der wenigstens ein Teil des Tragarms gestaltet ist, um bei Benutzung einen Platz für wenigstens einen der Finger des Benutzers zu schaffen.

12. Schablone nach einem der Ansprüche 1 bis 12, bei der ein oder mehrere Handgriffe vorhanden sind.

13. Schablone nach Anspruch 1, bei der das Betätigungsmittel ein mechanisches Mittel ist.

14. Schablone nach Anspruch 1, bei der das Applikatormittel mit Gewinde versehen ist und auf dem statischen Teil damit zusammenwirkende Gewindegänge vorgesehen sind.

15. Schablone nach Anspruch 1, bei der das Betätigungsmittel eine Anordnung mit Zahnstangengetriebe ist.

16. Schablone nach Anspruch 1, bei der das Betätigungsmittel ein Auslösemechanismus ist.

17. Schablone nach einem der Ansprüche 1 bis 16, bei der das Verbindermittel durch die Form des Endes des Applikatorteils gebildet ist.

18. Schablone nach einem der Ansprüche 1 bis 16, bei der das Verbindermittel ein auf dem distalen Ende des Applikatorteils befindlicher Kolben ist.

19. Schablone nach Anspruch 18, bei der der Kolben aus einem Kunststoffmaterial gebildet ist.

20. Schablone nach einem der Ansprüche 1 bis 19, bei der der Tragarm so ausgebildet ist, daß die Backe mit dem Femurhals zusammenwirkt.

21. Schablone nach einem der Ansprüche 1 bis 19, bei der der Tragarm so ausgebildet ist, daß die Backe mit dem Femurhals zusammenwirkt.

## Revendications

1. Gabarit (20) pour l'application d'une prothèse de resurfaçage de tête fémorale (31) sur une tête fémorale préparée dans lequel le gabarit comprend :
au moins un bras de support présentant une extrémité proximale (23), une extrémité distale (22) et un plan défini dans celui-ci ;
une mâchoire située à l'extrémité distale (22) du bras de support et définissant un plan, dans lequel le plan de la mâchoire est incliné par rapport au plan du bras de support ; et
un élément statique situé à l'extrémité proximale du ou de chaque bras de support ;
**caractérisé en ce qu'**il comprend en outre
un élément applicateur (26) associé à l'élément statique et déplaçable par rapport à celui-ci ; et
un élément connecteur (28) pour permettre à l'élément applicateur (26) durant l'utilisation d'interagir avec une surface externe d'une prothèse de resurfaçage de tête fémorale (31),
l'élément applicateur comprenant un moyen d'actionnement pour permettre le déplacement de l'élément applicateur (26) d'une première position supérieure à une seconde position inférieure.

2. Gabarit selon la revendication 1, dans lequel la mâchoire est une bague.

3. Gabarit selon la revendication 1 ou la revendication 2, dans lequel la mâchoire est située de façon à être sensiblement perpendiculaire au bras de support.

4. Gabarit selon la revendication 2, dans lequel la mâchoire est positionnée de telle sorte que le centre de l'élément applicateur soit aligné avec le point central de la bague.

5. Gabarit selon l'une quelconque des revendications 1 à 4, dans lequel le gabarit comporte de plus un collier détachable.

6. Gabarit selon la revendication 5, dans lequel le collier est une bague fendue.

7. Gabarit selon l'une quelconque des revendications 1 à 6, dans lequel l'élément statique situé à l'extrémité proximale du ou de chaque bras de support est un collier dans lequel l'élément applicateur est déplaçable.

8. Gabarit selon la revendication 7, dans lequel l'élément applicateur est une pièce à ajustement par coulissement dans le collier.

9. Gabarit selon la revendication 8, dans lequel l'extrémité supérieure de l'élément applicateur est munie d'un moyen d'actionnement pour permettre l'enfoncement de l'élément applicateur.

10. Gabarit selon la revendication 9, dans lequel le moyen d'actionnement est une plaque.

11. Gabarit selon la revendication 9 ou 10, dans lequel au moins une partie du bras de support est conformée pour servir de position à au moins l'un des doigts de l'utilisateur durant l'utilisation.

12. Gabarit selon l'une quelconque des revendications 1 à 12, présentant une ou plusieurs poignées.

13. Gabarit selon la revendication 1, dans lequel le moyen d'actionnement est un moyen mécanique.

14. Gabarit selon la revendication 1, dans lequel le moyen d'actionnement est fileté et des filets coopérants sont fournis sur l'élément statique.

15. Gabarit selon la revendication 1, dans lequel le moyen d'actionnement est un agencement à crémaillère.

16. Gabarit selon la revendication 1, dans lequel le moyen d'actionnement est un mécanisme de déclenchement.

17. Gabarit selon l'une quelconque des revendications 1 à 16, dans lequel le moyen connecteur est formé par la forme de l'extrémité de l'élément applicateur.

18. Gabarit selon l'une quelconque des revendications 1 à 16, dans lequel le moyen connecteur est un poussoir situé sur l'extrémité distale du moyen applicateur.

19. Gabarit selon la revendication 18, dans lequel le poussoir est formé en une matière plastique.

20. Gabarit selon l'une quelconque des revendications 1 à 19, dans lequel le bras de support est configuré de telle sorte que la mâchoire interagisse avec le col fémoral.

21. Gabarit selon l'une quelconque des revendications 1 à 19, dans lequel le bras de support est configuré de telle sorte que la mâchoire interagisse avec le col fémoral.
